# EUROPEAN PATENT APPLICATION

(11) **EP 4 432 294 A1**
(43) Date of publication of application: **18.09.2024**
(21) Application number: 23162094.9
(22) Date of filing: 15.03.2023
(51) Int. Cl.: G16H 15/00, G06N 20/00, G10L 15/00, G16H 40/20, G16H 40/40, G16H 70/20

(54) **METHOD FOR AUTOMATICALLY DOCUMENTING A LABORATORY WORKFLOW**

(71) Applicant: Labtwin GmbH, 10405 Berlin (DE)
(72) Inventor: Kulessa, Jonas, 10347 Berlin (DE); Gloth, Steffen, 10245 Berlin (DE); Thillainadarasan, Sukanija, 13599 Berlin (DE); Ballentin, Sven, 12435 Berlin (DE)
(74) Representative: Gottschald Patentanwälte Partnerschaft mbB

(57) **Abstract**

The invention relates to a method for automatically documenting a laboratory workflow via a control system (1), wherein the laboratory workflow is performed by a first user (2) inside a laboratory, wherein the laboratory comprises different laboratory devices (3) used for the laboratory workflow, wherein the control system (1) receives unstructured workflow data (4) from the different laboratory devices (3), wherein the unstructured workflow data (4) comprises documenting information about the performance of the laboratory workflow, wherein the control system (1) automatically documents the laboratory workflow by filling out a predefined template, wherein the template comprises data fields for different classes of data, wherein the control system (1) comprises a classification engine (5), wherein the classification engine (5) comprises one or more trained AI models (6), wherein the classification engine (5) receives the unstructured workflow data (4), wherein the classification engine (5) applies the one or more trained AI models (6) to analyze the unstructured workflow data (4), to extract the documenting information and to classify the documenting information into a group of classes including the classes of data of the template, wherein one of the one or more trained AI model (6) outputs classification certainties describing a probability of the classification being correct for each class, wherein the control system (1) fills out the data fields of the template by inputting the extracted documenting information into the data fields based on matching the classification with the classes of data of the template if the classification certainty for a respective class is above a predefined threshold, wherein, if the classification certainty for a respective data field is not above the predefined threshold, the control system (1) prompts a second user (8) to classify the documenting information.

## Description

The present invention relates to a method for automatically documenting a laboratory workflow according to claim 1 and to a control system adapted to perform the method according to claim 20.

In today's laboratory environments, there are high requirements for accuracy, precision, and reproducibility in the execution of laboratory processes. Additionally, there are high requirements for documentation of highly variable experimental workflows.

In this context, various assistance systems for supporting laboratory personnel have become known which can be summarized under the abbreviation LIMS (laboratory information and management system) or under the abbreviation ELN (electronic laboratory notebook). These are software-based data processing systems which support laboratory personnel with the provision and processing of laboratory data during a laboratory process. The known LIMS or ELN systems hardly go beyond the digitization of traditional paper-based laboratory documentation; thus, increasing efficiency when executing laboratory processes is possible only to a limited extent.

Many experimental workflows are documented via templates. The templates may depend on the experiment, the laboratory, the user and generally differ in many aspects. Filling out the templates is generally a time-consuming task that's hard to automate.

It is a challenge to improve on the known prior art.

The invention is based on the problem of improving the known methods for filling templates such that a further optimization is reached.

The above-noted object is solved by the features of claim 1.

The main realization of the present invention is that templates can be filled out automatically. However, automatic filling of templates may run into problems in some cases. Reverting those problems to the user will lead to problems with the acceptance of a system. The user needs to learn how to deal with the problems and divert their attention away from the laboratory work they are doing. If however the problems are not directed to the user in the laboratory, but to a specialist instead, the specialist can deal with the cases where the automation is not working properly in a standardized manner and based on experience. The results from this second user can then be used to improve the automatic system towards a full automation of the template filling.

In detail, proposed is a method for automatically documenting a laboratory workflow via a control system, wherein the laboratory workflow is performed by a first user inside a laboratory, wherein the laboratory comprises different laboratory devices used for the laboratory workflow, wherein the control system receives unstructured workflow data from the different laboratory devices, wherein the unstructured workflow data comprises documenting information about the performance of the laboratory workflow, wherein the control system automatically documents the laboratory workflow by filling out a predefined template, wherein the template comprises data fields for different classes of data, wherein the control system comprises a classification engine, wherein the classification engine comprises one or more trained Al models, wherein the classification engine receives the unstructured workflow data, wherein the classification engine applies the one or more trained AI models to analyze the unstructured workflow data, to extract the documenting information and to classify the documenting information into a group of classes including the classes of data of the template, wherein one of the one or more trained AI model outputs classification certainties describing a probability of the classification being correct for each class, wherein the control system fills out the data fields of the template by inputting the extracted documenting information into the data fields based on matching the classification with the classes of data of the template if the classification certainty for a respective class is above a predefined threshold, wherein, if the classification certainty for a respective data field is not above the predefined threshold, the control system prompts a second user to classify the documenting information.

In some embodiments according to claim 2, the unstructured workflow data may comprise measurements, experimental data and spoken user inputs. A laboratory generates a lot of data. By contextualizing the data and extracting the relevant aspects for the documentation, in particular in combination with voice commands, the templates can be filled with a high efficiency. By including the second user in the loop, the overall result can be optimized.

Embodiments according to claim 3 relate to the predefined threshold which in particular may be adjustable to take into account personal preferences and find a good compromise between effort and result.

According to claim 4, uncertainty levels may be attached to data in the unstructured workflow data. The uncertainty level can represent how reliable data from a certain source usually is and/or how reliable data from a certain source is usually correctly classified, for example. The uncertainty levels are therefore a way of including further knowledge about the data into the evaluation of the classification.

The embodiment according to claim 5 relates to receiving feedback from the second user and using this feedback to fill out the template.

Embodiments according to claim 6 relate to details about the second user who may be located at a central classification center at a remote location.

It may be the case that the group of classes includes classes not included in the template (claim 7). By not trying to force all data into the classes of a given template, the overall accuracy can be improved. Data not fitting into the template can then be handled or reclassified separately.

The control system may also find duplicate data and data that does not seem to fit into the context of the other data and send this data to the second user to check the data (claim 8).

The feedback from the second user may, in an embodiment according to claim 9, be used to improve the control system, in particular an Al model.

The control system may also use the feedback from the second user to update the template in an embodiment according to claim 10. This type of full service approach by an interaction of AI and the human second user allows the first user to document their laboratory workflows like experiments with low effort even if the template is not yet fully capable of handling all documentation.

In embodiments according to claim 11 the first user may sign off the template at the end. Further, the first user and/or the control system may choose the template at the beginning.

In embodiments according to claim 12, the classification certainty is sent to the second user who may use it to better derive the correct classification and/or actions and work more efficiently.

Claim 13 names different laboratory devices that may be sources for the unstructured workflow data.

Claims 14 to 19 relate to preferred aspects of using speech recognition and improving the speech recognition by using the templates. Generally, the accuracy of speech models depends on a number of factors, many of which can be eliminated, or their influence reduced by using the context-knowledge from the template filling.

In an embodiment according to claim 15, the speech model is fine-tuned. According to claim 16, the speech recognition may start with a standard model and the standard model may be improved, in particular over time, to adapt to the specifics of the first user and/or the laboratory and/or the template.

Claims 17 and 18 detail further embodiments for improving the speech model.

According to claim 19, the speech recognition may be done in two steps by first recognizing some of the speech, then recognizing the context via the templates and then improving the speech recognition, for example by redoing it with a more specialized speech model.

Another teaching according to claim 20, which is of equal importance, relates to a control system adapted to perform the method according to one of the preceding claims.

All explanations given with regard to the proposed method are fully applicable.

In the following, embodiments of the invention are explained with respect to the drawing. The drawing shows in
- Fig. 1,: a high level overview of the control system in use and
- Fig. 2,: a flow chart detailing an embodiment of the proposed method.

Proposed is a method for automatically documenting a laboratory workflow via a control system 1. The control system 1 may comprise local hardware components and/or cloud-based components.

The proposed method serves to support workflows in a laboratory environment. The laboratory workflow is preferably a workflow for performing an experiment, in particular a bioprocess, which includes also production processes and their good manufacturing practice (GMP) requirements.

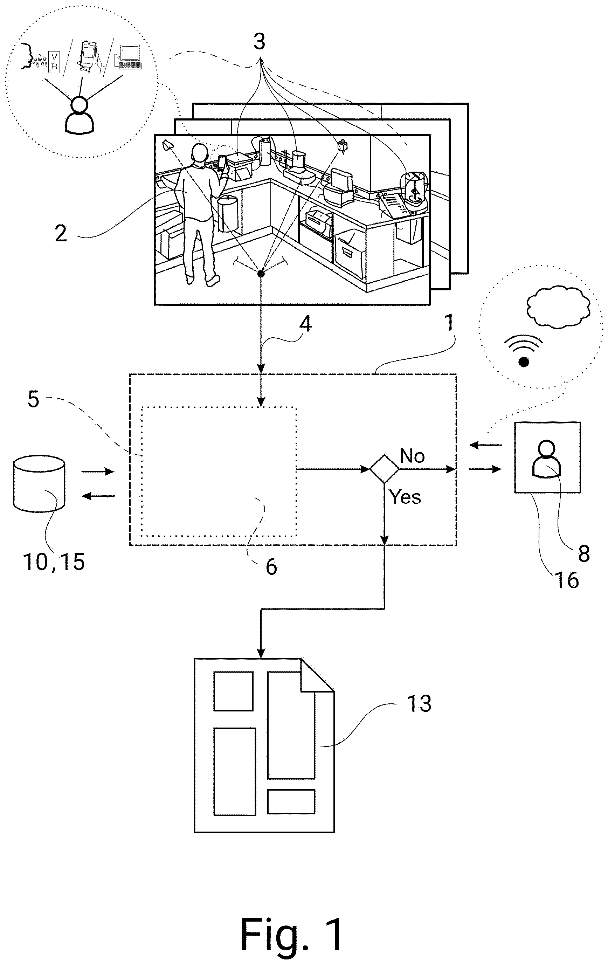

The laboratory workflow is performed by a first user 2 inside a laboratory, exemplarily shown in Fig. 1. The laboratory comprises different laboratory devices 3 used for the laboratory workflow.

The control system 1 receives unstructured workflow data 4 from the different laboratory devices 3. The unstructured workflow data 4 is data generated in context with the performance of the workflow, for example measurements and the like. The unstructured workflow data 4 is generated by the laboratory devices 3. The control system 1 receives the unstructured workflow data 4 directly from the laboratory devices 3 and/or indirectly through a laboratory management system. Some of the unstructured workflow data 4 may also be input into the control system 1 by the first user 2 for example in the evening. Time stamps may then be used to relate the data to other data of the day.

The unstructured workflow data 4 comprises documenting information about the performance of the laboratory workflow, for example steps performed, substances used, substances combined, laboratory devices 3 used, measurements taken, experiments and workflow steps performed and so on.

The control system 1 automatically documents the laboratory workflow by filling out a predefined template. The template may have been predefined by the first user 2, the organization of the first user 2, or the like. The template comprises data fields for different classes of data. Exemplarily, a template can include a group of data fields for a first experiment, a group of data fields for a second experiment, in particular including data fields describing the differences between the experiments, and so on. The first experiment and the second experiment may have at least partially data fields of the same class, such that a differentiation of the context is necessary.

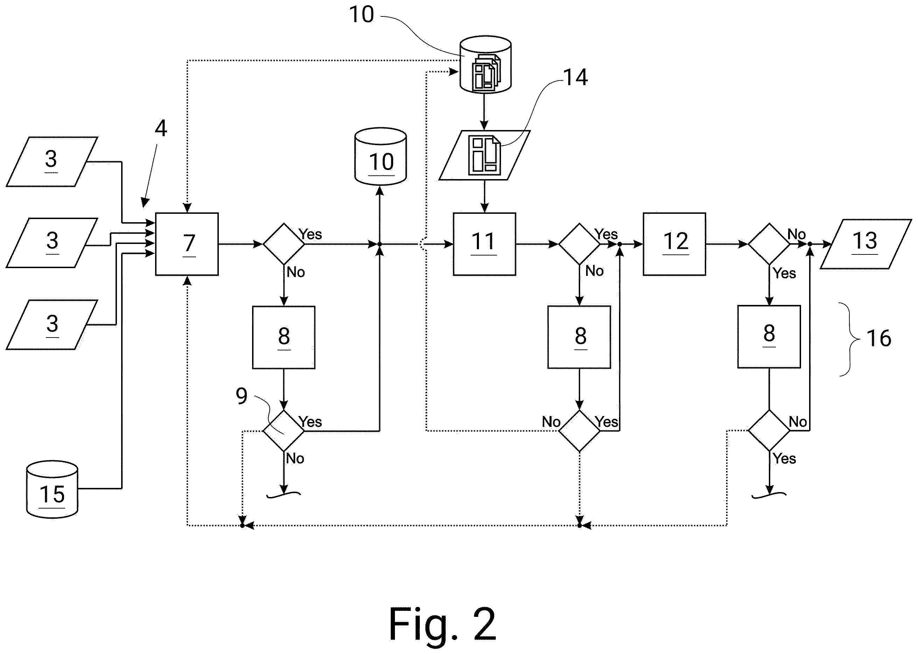

The control system 1 comprises a classification engine 5. The classification engine 5 comprises one or more trained AI models 6. Fig. 1 shows the unstructured workflow data 4 being sent to the control system 1 and entered into the classification engine 5. Fig. 2 shows what might happen inside the control system 1 and classification engine 5 in form of an abstract flow chart.

The classification engine 5 receives the unstructured workflow data 4. The classification engine 5 applies the one or more trained AI models 6 to analyze the unstructured workflow data 4, to extract the documenting information and to classify the documenting information into a group of classes including the classes of data of the template. The group of classes and the classes of data of the template have some overlap. In many cases the group of classes is selected and/or adapted based on the template, in particular have a higher overlap. However, some classes of the template and/or of the group of classes may have no match in the other group. This case can be handled separately as will be described.

Naturally, any classification has a certain probability of being incorrect. One of the one or more trained AI models 6 outputs classification certainties describing a probability of the classification being correct for each class. In Fig. 2, behind the classification engine 5 a check of the classification certainties 7 for each classification is implemented. If for example a classification certainty is, already at this step, below a threshold, a second user 8 may be prompted to reclassify the data. The user may decide at a reclassification decision step 9 to reclassify the data or that possibly the extracted data is invalid or the like. The classification may be stored inside the database 10. Then, the method may go on towards the next step.

The control system 1 fills out the data fields of the template by inputting the extracted documenting information into the data fields based on matching the classification with the classes of data of the template if the classification certainty for a respective class is above a predefined threshold. Fig. 2 shows a matching step 11 in which the group of classes is matched onto the classes of the template. This matching may have a further uncertainty if classes are similar, for example. This further uncertainty can be included in the classification certainty, for example by multiplication. The classification certainty therefore is the certainty of correctly classifying the data into the classes of the template, which happens in two steps here.

If the classification certainty for a respective data field is not above the predefined threshold, the control system 1 prompts a second user 8 to classify the documenting information. The second user 8 may then use a class of the template and do the above matching by hand or may change the template, as shown with the yes/no paths in Fig. 2. After the step of filling the template 12, the filled template 13 may also pass the second user 8 as shown in Fig. 2 before becoming the final filled template 13. Fig. 1 shows the decisions of Fig. 2 only aggregated into a single decision step.

Different types of data may also influence the path of the data. For example, a barcode reading may have such a high classification certainty that it always goes into the template directly without passing the second user 8.

Fig. 2 shows a number of empty templates 14 from which one is chosen. Fig. 1 shows a filled template 13. Both are shown only schematically.

One of the one or more AI models may be or comprise a large language model.

To explain the details of the proposed method, the following abstract example will guide through a possible workflow.

Before the laboratory workflow is started, the first user 2 creates a template in a web application, uploads an existing template or selects one from a database 10. In a next step the first user 2 assigns the template to the workflow that is to be run. Within the beginning of the laboratory workflow, the assigned template is started to be filled out until the workflow is ended, wherein the workflow may comprise a plurality of experiments and sub-workflows.

More precisely, while the workflow takes place, various unstructured workflow data 4 is generated and recorded by the laboratory devices 3. As an example, a voice notice from a first user 2 is recorded by a laboratory device 3, here a microphone. The voice notice, as unstructured workflow data 4, may contain the note of the first user 2 that a color change of a certain sample was observed within a particular experiment that is part of the workflow. This color change may be the documenting information to be extracted and to be inserted into a "color" data field of the template.

Incoming unstructured workflow data 4 can be linked into groups, where this data can also include data groups or subgroups. The linking of data can be predefined, for example by templates, data banks or standard operating procedures (SOPs). Likewise, the linking of data pairs can be done by the control system 1 as part of the classification, in particular by the classification engine 5 and a trained AI model 6 or the second user 8. Both are not mutually exclusive and will complement each other in some cases in a way which leads to a continuous improvement of the performance. For this, improvement of the entire system is possible through suitable updates, e.g. of the AI or the template, based on the decisions made.

In some cases, especially with complex workflow data such as voice notes or images, additional workflow data can be extracted from raw workflow data and will be linked to it in the described way. An example may be, when the first user 2 mentions in his note also the name of the sample and the type of the experiment. In other cases, some workflow data requires the interaction with the user. For example, it is conceivable to actively ask the user for a specific data point in a pre-defined workflow (e.g. "What is the color of sample A?"), particularly for critical data.

The linking of the data may include adding metadata to other data. The metadata may for example include data from barcode scans or a database 10.

As part of the classification the documenting information will be classified into defined classes of data. Practically, this can mean that the mentioned voice notice about the observed color change, is assigned to the class observations, for example. If the thereby resulting classification certainty for a respective class is above the predefined threshold, the documentation data is processed further. If this is not the case, the second user 8 is consulted. Decisions made by the second user 8 can be used as feedback and in form of an update to further optimize the trained AI and the template.

During this processing step, the system can also standardize values. This can concern the units, but also different terms with the same meaning. It becomes especially relevant when users speak different languages.

The further processing of the documenting data comprises a matching step 11 with the assigned template, based on the respective classification. For example, the further mentioned voice notice classified as an observation is assigned to a corresponding data field in the template according to its context, if available. If matching, in this example, is not possible because the template does not provide for the entry of observations so far, a higher instance in the form of the second user 8, is consulted. Decisions made here have an analogous influence on the further development of the template, the template database 10 and the trained AI model 6 via resulting in respective updates.

Within the framework of the described data processing the control system 1 may also detect duplicates and inconsistencies, send them in the previous described manner to the second user 8 and integrate the decisions in the same way in an update process to improve the system successively. In some embodiments, this can also include checking whether value lies within a certain range, matching a specified data type (e.g. a number) or a specific format. This verification routine can in principle be applied at different points in the process.

It is conceivable, that also unclassified data, unverified data or even data fragments are appropriately documented, for example in databases 10, so that in principle nothing is lost, even if this data is at the moment not relevant for filling out the selected template.

The here exemplary described method with an automatic template filling of the template results in comprehensive documentation of the laboratory workflow. After completion of the workflow, the documents can be countersigned by the first and the second user 8 if specified in related guidelines like SOPs. The system generates a report as suitable output. That can mean that a structured and machine-readable data object will be provided or for example a final formatted report, preferably with visualizations, is available in an appropriate data-format such as a PDF file.

According to one embodiment it is proposed, that the unstructured workflow data 4 comprises measurements, preferably laboratory device 3 data, and/or, experimental result data, and/or user inputs. The user inputs may be inputs written on a computer and/or recorded spoken user input, for example.

Preferably, the control system 1 transcribes the spoken user input and includes the transcribed spoken user input in the unstructured workflow data 4. Preferably, the control system 1 removes the spoken user input from the unstructured workflow data 4 afterwards and before inputting it into the classification engine 5.

The unstructured workflow data 4 may comprise data of laboratory device 3 like a device identifier, e.g., a barcode ID, general device information, e.g., a location, a configuration, a type of laboratory device 3 and/or sensor data from a laboratory device 3.

The unstructured workflow data 4 may comprise experimental result data, like analysis results obtained from a laboratory device 3, for example numerical data, e.g., concentrations, intensity values, fluorescence values, photo data, e.g., microscopic images, video data and/or metadata, e.g., a temperature of a HPLC column, like user data, e.g., a user ID of a user who performed an analysis and/or like protocol information, e.g., which protocol was used for an analysis, a time of an experiment, etc.

Other data like data from external sensors, e.g., temperature or humidity in the laboratory, SOP information, text notes by users and/or images/videos taken by a user may also be included in the unstructured workflow data 4. Further data may stem from a company database 15.

It may be the case that the predefined threshold is above 80%, preferably above 90%, more preferably above 95%. To adapt to the importance of a certain workflow and/or to personal preferences or for other reasons the predefined threshold may be adjusted by the first user 2 and/or the second user 8.

If the certainty is below the threshold, the certainty can also be used to guide the second user 8, for example by providing additional information for decision-making, e.g., by displaying only the 5 most probable classifications that should go to the selected field of the template.

As already discussed, it may be the case that the control system 1 assigns uncertainty levels to the unstructured workflow data 4, in particular based on their source, preferably, that the uncertainty levels are included in the unstructured workflow data 4. The control system 1 may correct the classification certainties based on the uncertainty levels. As mentioned above, barcodes may provide a high certainty while data from a video analysis of a camera may have a very low certainty. Some type of data may even always go to the second user 8 until the control system 1 has been adapted to the workflow and/or template and/or first user 2 and/or laboratory.

According to one embodiment it is proposed, that the control system 1 receives a classification of the documenting information from the second user 8 and inputs the documenting information into the data fields based on matching the classification with the classes of data of the template. This may either be the step after the reclassification decision step 9 or the intervention of the second user 8 behind the matching step 11 in Fig. 2.

Here and preferably the second user 8 is different from the first user 2. It may be the case that the control system 1 sends the documenting information with the classification certainty below the predefined threshold to a central classification system 16 at a location remote to the laboratory and that the central classification system 16 outputs the documenting information to the second user 8 for classification. Preferably, the central classification system 16 receives documenting information from multiple control systems 1 at different laboratories. The control system 1 and the central classification system 16 may communicate over the internet or run in the same cloud environment, for example in separate instances.

According to one embodiment it is proposed, that the group of classes comprises classes not included in the classes of data of the template. Preferably, data classified into one of the classes not included in the classes of data of the template with a classification certainty above the predefined threshold is appended to the template, and/or, data classified into one of the classes not included in the classes of data of the template with a classification certainty above the predefined threshold is sent to the second user 8 to derive an action for said data. It is preferably so that not all data is classified into only classes of the template. It may even be the case that the group of classes is independent of the template and a further layer, which may be improved over time, matches the group of classes with the classes of the template. For example, the group of classes may be a huge group of classes, many of them being assigned to a single class in the template.

It is also possible that the control system 1 detects documenting information that is duplicate and/or documenting information that does not fit other data in the documenting information and sends this documenting information to the second user 8 to derive an action for said data.

According to one embodiment it is proposed, that the control system 1, in particular at least one of the one or more trained AI models 6, is updated based on the classifications and/or actions of the second user 8.

According to another embodiment it is proposed, that the template is updated based on the classifications and/or actions of the second user 8.

Here and preferably the first user 2 signs off the filled out template. The first user 2 will usually be the best person to judge whether the template has been filled correctly. Additionally or alternatively, the first user 2 selects a template to be filled out. Alternatively, the control system 1 selects a template to be filled out based on the documenting information, in particular based on the classes the documenting information is classified into.

As mentioned it may be the case that the classification certainty is sent to the second user 8 with the documenting information and/or included in the template. The latter allows judging a template later on if an information in the template does not seem to be correct. Further context may be available in the control systems 1 database 10 then.

According to one embodiment it is proposed, that the different laboratory devices 3 comprise a camera and/or a microphone and/or a scanner and/or a bar code scanner and/or laboratory equipment, preferably, that the laboratory equipment includes a scale and/or a temperature probe and/or a fluid analyzer.

The laboratory devices 3 may comprise analysis devices, e.g., a HPLC device, a plate reader, a microscope, an NMR device, a pH measurement device, an osmolality measurement device, a photometer and so on. The laboratory devices 3 may comprise liquid handling systems like robotic systems for sample dilution, sample transfer, etc. and auxiliary devices, e.g., centrifuges, incubators, shakers, thermocyclers, microscopes, etc. and cultivation/purification devices, e.g., bioreactors, chromatography systems, filtration systems etc.

Turning towards the spoken user input, it is possible that the control system 1 receives the spoken user input from the first user 2 and that the control system 1 performs a speech recognition on the spoken user input, in particular to transcribe the spoken user input, using a speech model, in particular a trained AI speech model, to detect words spoken by the user from the spoken user input. Spoken user input is audio data recorded by a microphone with spoken words, phrases, sentences and possibly noise. This spoken input is converted into text to be fed for example into a large language model. The conversion into text is a first mayor source of uncertainty that should be tackled.

The control system 1 may generate and/or adapt and/or choose the speech model based on data relating to the template, in particular based on classes of the template. By using a speech model that fits the current context, which is well described by the template that in turn may be strongly related to the workflow, the speech recognition may be enhanced. For example, the speech model may know the names and possibly manufacturers of devices used in the workflow. The speech model may also use usual "slang" in the field. The speech model can be generated or adapted from a standard model or several models may be stored in the database 10, possibly linked to the templates.

According to one embodiment it is proposed, that the trained AI speech model is fine-tuned with training data including the classifications from the second user 8. Fine-tuning is a well known process in the field of AI to improve a model trained on a large general data pool with few specific training data. Additionally or alternatively, the control system 1 may adapt the speech model based on the classifications from the second user 8. After a number of classifications from the second user 8, the speech model may be, in particular automatically, fine-tuned further. It may also be the case that a new or newly generated or newly adapted speech model is used in parallel with the previous speech model for a time. The new model can then be compared to the old model and swapped if the output of the new model is better.

The control system 1 may use a standard speech model and adapt the standard speech model based on the template, in particular to better recognize data expected in the context of the template. Preferably, the control system 1 adapts weights of word and/or phrases based the template. The weights may be probabilities of occurrences of words. If a spoken input has a high probability of either being laboratory or Labrador, for example, the word laboratory may be weighed higher in the current context and may therefore be chosen, even if the word sounded more like Labrador for the speech model.

According to one embodiment it is proposed that, if the classification certainty for a respective data field originating from the spoken user input is not above the predefined threshold, the control system 1 adapts the speech model for future speech recognition. The speech model does not have to be adapted directly, the respective case may be entered into training data for future training of the model.

Preferably, the control system 1 adapts the speech model based on a context in the laboratory and the classifications from the second user 8 and uses the adapted speech model in a similar context in the future. In that way, for example, a preprocessing of noise may be changed or a certain slang of a certain laboratory may be learned. The similar context may include the same user, the same laboratory, the same workflow, the same company, the same geographical region, the same template and the like.

It may also be the case that the control system 1 sends speech input with a recognition probability below a threshold to the second user 8, that the control system 1 receives from the second user 8 a recognition, that the control system 1 links the received recognition with the template, that the control system 1 uses terms linked with the template to adapt the speech model. For example, the terms linked with the template may be weighed higher as explained above.

According to one embodiment it is proposed, that the control system 1 performs a two-step speech recognition on the spoken user input, that the control system 1 in a first step recognizes at least some of the speech using a first speech model, that the control system 1 then applies the one or more trained AI models 6 to classify at least some of the recognized speech, that the control system 1 generates and/or chooses a further speech model and/or adapts the first speech model into a further speech model based on the result of the classification, and in particular based on the template, preferably by identifying a context based on matching the classification with the template, that the control system 1 in a second step recognizes at least some other part of the speech and/or improves the recognition of the speech from the first step using the further speech model. For example, an unknown voice recording may be analyzed with a high rate of errors. The control system 1 may be able to recognize a certain template as being a probable target of the voice recording. The control system 1 may then select a speech model related to the template and do another speech recognition on the voice recording, this time recognizing the content better.

Another teaching which is of equal importance relates to a control system 1 adapted to perform the proposed method according to one of the preceding claims. All explanations given with regard to the proposed method are fully applicable.

## Claims

1. Method for automatically documenting a laboratory workflow via a control system (1), wherein the laboratory workflow is performed by a first user (2) inside a laboratory, wherein the laboratory comprises different laboratory devices (3) used for the laboratory workflow, wherein the control system (1) receives unstructured workflow data (4) from the different laboratory devices (3), wherein the unstructured workflow data (4) comprises documenting information about the performance of the laboratory workflow,
wherein the control system (1) automatically documents the laboratory workflow by filling out a predefined template, wherein the template comprises data fields for different classes of data,
wherein the control system (1) comprises a classification engine (5), wherein the classification engine (5) comprises one or more trained AI models (6), wherein the classification engine (5) receives the unstructured workflow data (4), wherein the classification engine (5) applies the one or more trained AI models (6) to analyze the unstructured workflow data (4), to extract the documenting information and to classify the documenting information into a group of classes including the classes of data of the template, wherein one of the one or more trained AI model (6) outputs classification certainties describing a probability of the classification being correct for each class,
wherein the control system (1) fills out the data fields of the template by inputting the extracted documenting information into the data fields based on matching the classification with the classes of data of the template if the classification certainty for a respective class is above a predefined threshold,
wherein, if the classification certainty for a respective data field is not above the predefined threshold, the control system (1) prompts a second user (8) to classify the documenting information.

2. Method according to claim 1, **characterized in that** the unstructured workflow data (4) comprises measurements, preferably laboratory device (3) data, and/or, experimental result data, and/or, user inputs, preferably recorded spoken user input, preferably, that the control system (1) transcribes the spoken user input and includes the transcribed spoken user input in the unstructured workflow data (4), more preferably, that the control system (1) removes the spoken user input from the unstructured workflow data (4).

3. Method according to claim 1 or 2, **characterized in that** the predefined threshold is above 80%, preferably above 90%, more preferably above 95%, preferably, that the predefined threshold can be adjusted by the first user (2) and/or the second user (8).

4. Method according to one of the preceding claims, **characterized in that** the control system (1) assigns uncertainty levels to the unstructured workflow data (4), in particular based on their source, preferably, that the uncertainty levels are included in the unstructured workflow data (4), and/or, that the control system (1) corrects the classification certainties based on the uncertainty levels.

5. Method according to one of the preceding claims, **characterized in that** the control system (1) receives a classification of the documenting information from the second user (8) and inputs the documenting information into the data fields based on matching the classification with the classes of data of the template.

6. Method according to one of the preceding claims, **characterized in that** the second user (8) is different from the first user (2), preferably, that the control system (1) sends the documenting information with the classification certainty below the predefined threshold to a central classification system (16) at a location remote to the laboratory, that the central classification system (16) outputs the documenting information to the second user (8) for classification, preferably, that the central classification system (16) receives documenting information from multiple control systems (1) at different laboratories.

7. Method according to one of the preceding claims, **characterized in that** the group of classes comprises classes not included in the classes of data of the template, preferably, that data classified into one of the classes not included in the classes of data of the template with a classification certainty above the predefined threshold is appended to the template, and/or, that data classified into one of the classes not included in the classes of data of the template with a classification certainty above the predefined threshold is sent to the second user (8) to derive an action for said data.

8. Method according to one of the preceding claims, **characterized in that** the control system (1) detects documenting information that is duplicate and/or documenting information that does not fit other data in the documenting information and sends this documenting information to the second user (8) to derive an action for said data.

9. Method according to one of the preceding claims, **characterized in that** the control system (1), in particular at least one of the one or more trained AI models (6), is updated based on the classifications and/or actions of the second user (8).

10. Method according to one of the preceding claims, **characterized in that** the template is updated based on the classifications and/or actions of the second user (8).

11. Method according to one of the preceding claims, **characterized in that** the first user (2) signs off the filled out template, and/or that the first user (2) selects a template to be filled out, or, that the control system (1) selects a template to be filled out based on the documenting information, in particular based on the classes the documenting information is classified into.

12. Method according to one of the preceding claims, **characterized in that** the classification certainty is sent to the second user (8) with the documenting information and/or included in the template.

13. Method according to one of the preceding claims, **characterized in that** the different laboratory devices (3) comprise a camera and/or a microphone and/or a scanner and/or a bar code scanner and/or laboratory equipment, preferably, that the laboratory equipment includes a scale and/or a temperature probe and/or a fluid analyzer.

14. Method according to one claims 2 to 13, **characterized in that** the control system (1) receives the spoken user input from the first user (2), that the control system (1) performs a speech recognition on the spoken user input, in particular to transcribe the spoken user input, using a speech model, in particular trained AI speech model, to detect words spoken by the user from the spoken user input, that the control system (1) generates and/or adapts and/or chooses the speech model based on data relating to the template, in particular based on classes of the template.

15. Method according to claim 14, **characterized in that** the trained AI speech model is fine-tuned with training data including the classifications from the second user (8), and/or, that the control system (1) adapts the speech model based on the classifications from the second user (8).

16. Method according to claim 14 or 15, **characterized in that** the control system (1) uses a standard speech model and adapts the standard speech model based on the template, in particular to better recognize data expected in the context of the template, preferably, that the control system (1) adapts weights of word and/or phrases based the template.

17. Method according to one of claims 14 to 16, **characterized in that**, if the classification certainty for a respective data field originating from the spoken user input is not above the predefined threshold, the control system (1) adapts the speech model for future speech recognition, preferably, that the control system (1) adapts the speech model based on a context in the laboratory and the classifications from the second user (8) and uses the adapted speech model in a similar context in the future.

18. Method according to one of claims 14 to 17, **characterized in that** the control system (1) sends speech input with a recognition probability below a threshold to the second user (8), that the control system (1) receives from the second user (8) a recognition, that the control system (1) links the received recognition with the template, that the control system (1) uses terms linked with the template to adapt the speech model.

19. Method according to one of the preceding claims, **characterized in that** the control system (1) performs a two-step speech recognition on the spoken user input, that the control system (1) in a first step recognizes at least some of the speech using a first speech model, that the control system (1) then applies the one or more trained AI models (6) to classify at least some of the recognized speech, that the control system (1) generates and/or chooses a further speech model and/or adapts the first speech model into a further speech model based on the result of the classification, and in particular based on the template, preferably by identifying a context based on matching the classification with the template, that the control system (1) in a second step recognizes at least some other part of the speech and/or improves the recognition of the speech from the first step using the further speech model.

20. Control system adapted to perform the method according to one of the preceding claims.
